# EUROPEAN PATENT APPLICATION

(11) **EP 4 546 368 A1**
(43) Date of publication of application: **30.04.2025**
(21) Application number: 23826287.7
(22) Date of filing: 16.06.2023
(51) Int. Cl.: G16H 50/30

(54) **INFORMATION PROCESSING METHOD AND APPARATUS, WEARABLE DEVICE AND ELECTRONIC DEVICE**

(30) Priority: 23.06.2022 CN 202210719951
(71) Applicant: Vivo Mobile Communication Co., Ltd., Dongguan, Guangdong 523863 (CN)
(72) Inventor: LIU, Peng, Dongguan, Guangdong 523863 (CN)
(74) Representative: Lavoix
(86) International application number: PCT/CN2023/100692
(87) International publication number: WO 2023/246638

(57) **Abstract**

This application discloses an information processing method and apparatus, a wearable device, and an electronic device, which relate to the field of communication technologies. The information processing method includes: obtaining health data of a user; receiving time information sent by an electronic device in a case that a preset condition is met; and sending first information to the electronic device based on the time information, where the preset condition includes at least one following: a data volume of obtained health data being greater than or equal to a preset value, or a first instruction being obtained, where the first instruction is used for instructing to stop obtaining the health data; and the first information includes: storage position information of health data within a first time range and a data identifier of the health data, where the first time range is a time range corresponding to the time information; or the first information includes: the storage position information, the data identifier, a first moment, and a second moment, where the first moment is a moment at which the health data starts to be obtained, and the second moment is a moment at which the health data stops being obtained.

## Description

### CROSS-REFERENCE TO RELATED APPLICATIONS

This application claims priority to Chinese Patent Application No. 202210719951.9, filed in China on June 23, 2022, which is incorporated herein by reference in its entirety.

### TECHNICAL FIELD

This application relates to the field of communication technologies, and in particular, to an information processing method and apparatus, a wearable device, and an electronic device.

### BACKGROUND

Currently, with the development of technologies, monitoring and analysis of health data of a user can be implemented. Specifically, for example, a cardio pulmonary coupling (Cardio Pulmonary Coupling, CPC) analysis method can accurately monitor sleep and analyze a sleep cycle. Specifically, it generally requires a wearable device (such as a watch) in coordination with an electronic device (such as a mobile phone) for implementation. However, this solution has a problem of a long data processing delay time, which affects the user's use. For example, on an embedded low-power consumption watch, due to limitations of hardware performance and system power consumption, an algorithm needs to be integrated at a mobile phone. All ventricular beat interval (RRI) data is collected at the watch and then transmitted to the mobile phone for data analysis. After algorithm analysis at the mobile phone is successful, a result is sent back to the watch. Therefore, if the user views sleep data immediately after waking up, there is a delay in the display of sleep stages and scores on the watch and the mobile phone, which affects user experience.

From the above, the solution for processing such as analyzing and displaying the health data of a user in the related art has a problem of a long delay time.

### SUMMARY

An objective of embodiments of this application is to provide an information processing method and apparatus, a wearable device, and an electronic device, which can resolve a problem of a long delay time in a solution for processing health data of a user in the related art.

According to a first aspect, an embodiment of this application provides an information processing method, and the method includes:
obtaining health data of a user;
receiving time information sent by an electronic device in a case that a preset condition is met; and
sending first information to the electronic device based on the time information, where
the preset condition includes at least one of the following: a data volume of the obtained health data being greater than or equal to a preset value, or a first instruction being obtained, where the first instruction is used for instructing to stop obtaining the health data; and
in a case that the preset condition is a data volume of the obtained health data being greater than or equal to a preset value, the first information includes: storage position information of health data within a first time range and a data identifier of the health data, where the first time range is a time range corresponding to the time information; or
in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained.

According to a second aspect, an embodiment of this application provides an information processing method, and the method includes:
sending time information to a wearable device, where the time information is time range information used for obtaining health data of a user;
receiving first information sent by the wearable device based on the time information; and
performing a data processing operation based on the first information, where
the first information includes: storage position information of health data within a first time range and a data identifier of the health data, where the first time range is a time range corresponding to the time information; or
the first information includes: the storage position information, the data identifier, a first moment, and a second moment, where the first moment is a moment at which the health data starts to be obtained, and the second moment is a moment at which the health data stops being obtained.

According to a third aspect, an embodiment of this application provides an information processing apparatus, and the apparatus includes:
a first obtaining module, configured to obtain health data of a user;
a first receiving module, configured to receive time information sent by an electronic device in a case that a preset condition is met; and
a first sending module, configured to send first information to the electronic device based on the time information, where
the preset condition includes at least one of the following: a data volume of the obtained health data being greater than or equal to a preset value, or a first instruction being obtained, where the first instruction is used for instructing to stop obtaining the health data; and
in a case that the preset condition is a data volume of the obtained health data being greater than or equal to a preset value, the first information includes: storage position information of health data within a first time range and a data identifier of the health data, where the first time range is a time range corresponding to the time information; or
in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained.

Optionally, the obtaining health data of a user includes:
obtaining first health data and the difference between second health data and third health data;
storing the first health data and the difference, where
the first health data is health data obtained at the first moment;
the second health data is health data obtained at a third moment; and
the third health data is health data obtained at a fourth moment, where
the third moment is adjacent to the fourth moment, the third moment is within the first moment and the second moment, and the fourth moment is within the first moment and the second moment.

Optionally, the information processing apparatus further includes:
a first processing module, configured to restore the obtained health data based on the first health data and the difference after the time information sent by the electronic device is received;
a first determining module, configured to: determine health data within the first time range based on the restored health data; and
write the difference corresponding to the health data within the first time range and the first health data into a synchronization file, where
different synchronization files correspond to different storage position information.

Optionally, the determining the health data within the first time range based on the restored health data includes:
obtaining the restored health data within a target time range as the health data within the first time range, where
the target time range includes: a start moment of the first time range to an end moment of the first time range; or
a start moment of the first time range to a moment at which a last item is obtained, the last item is a last item of the obtained health data, and the moment at which a last item is obtained is earlier than the end moment of the first time range.

Optionally, the information processing apparatus further includes:
a second processing module, configured to: in a case that the preset condition is a data volume of the obtained health data being greater than or equal to a preset value, after the first information is sent to the electronic device based on the time information, recalculate a data volume of health data that continues to be obtained, and return to the receiving time information sent by an electronic device in a case that a preset condition is met.

Optionally, the first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and
the information processing apparatus further includes:
a third processing module, configured to: in a case that the preset condition is a first instruction being obtained, and the first instruction is an instruction triggered before the target event ends, after the first information is sent to the electronic device based on the time information, recalculate a data volume of health data that continues to be obtained, and return to the receiving time information sent by an electronic device in a case that a preset condition is met.

Optionally, the information processing apparatus further includes:
a second obtaining module, configured to: in a case that the preset condition is a first instruction being obtained, after the first information is sent to the electronic device based on the time information, obtain a time difference between a fifth moment and the second moment; and
a second sending module, configured to send the fifth moment to the electronic device in a case that the time difference is greater than a retransmission threshold, where
the fifth moment is an actual moment at which a target event corresponding to the health data ends, and the second moment is a reference moment at which the target event ends.

According to a fourth aspect, an embodiment of this application provides an information processing apparatus, and the apparatus includes:
a third sending module, configured to send time information to a wearable device, where the time information is time range information used for obtaining health data of a user;
a second receiving module, configured to receive first information sent by the wearable device based on the time information; and
a first execution module, configured to perform a data processing operation based on the first information, where
the first information includes: storage position information of health data within a first time range and a data identifier of the health data, where the first time range is a time range corresponding to the time information; or
the first information includes: the storage position information, the data identifier, a first moment, and a second moment, where the first moment is a moment at which the health data starts to be obtained, and the second moment is a moment at which the health data stops being obtained.

Optionally, the performing a data processing operation based on the first information includes:
obtaining health data of a user within the first time range based on the storage position information and the data identifier and storing the health data in a case that the first information includes the storage position information and the data identifier; or
obtaining health data of a user within the first time range based on the storage position information and the data identifier and storing the health data in a case that the first information includes the storage position information, the data identifier, the first moment, and the second moment;
splicing all the obtained health data corresponding to the data identifier; and
analyzing the spliced health data based on the first moment and the second moment.

Optionally, the health data includes: first health data and the difference between second health data and third health data, where the first health data is health data obtained at the first moment; the second health data is health data obtained at a third moment; and the third health data is health data obtained at a fourth moment, where the third moment is adjacent to the fourth moment, the third moment is within the first moment and the second moment, and the fourth moment is within the first moment and the second moment; and
the analyzing the spliced health data based on the first moment and the second moment includes:
restoring the spliced health data based on the first health data and the difference; and
analyzing the restored health data based on the first moment and the second moment.

Optionally, the first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and
the analyzing the spliced health data based on the first moment and the second moment includes:
analyzing the spliced health data based on the first moment and a target moment, where the target moment is a moment at which a target instruction is triggered, and the target instruction is a first instruction triggered when the target event ends; and
the performing a data processing operation based on the first information further includes:
   obtaining a health data of a user within the first time range based on the storage position information and the data identifier and storing the health data in a case that the first instruction is an instruction triggered before the target event ends.

Optionally, the analyzing the spliced health data based on the first moment and the second moment includes:
in a case that a fifth moment sent by the wearable device is not received after the first information is received, analyzing the spliced health data based on the first moment and the second moment; and
in a case that the fifth moment is received after the first information is received, analyzing the spliced health data based on the first moment and the fifth moment, where
the fifth moment is an actual moment at which a target event corresponding to the health data ends, and the second moment is a reference moment at which the target event ends.

Optionally, the sending time information to a wearable device includes:
using an end moment at which the health data is obtained in a historical record as a start moment at which the health data is obtained;
determining, based on a system moment, an end moment at which the health data is obtained, where the end moment is a moment before the system moment or the system moment;
obtaining, based on the start moment and the end moment, time information for obtaining health data of a user; and
sending the time information to the wearable device.

According to a fifth aspect, an embodiment of this application provides a wearable device, including a processor, a memory, and a program or instructions stored on the memory and executable on the processor, where the program or the instructions, when executed by the processor, implement the steps of the method according to the first aspect.

According to a sixth aspect, an embodiment of this application provides an electronic device, including a processor, a memory, and a program or instructions stored on the memory and executable on the processor, where the program or the instructions, when executed by the processor, implement the steps of the method according to the second aspect.

According to a seventh aspect, an embodiment of this application provides a readable storage medium, having a program or instructions stored therein, where the program or instructions, when executed by a processor, implement the steps of the method according to the first aspect or the second aspect.

According to an eighth aspect, an embodiment of this application provides a chip, including a processor and a communication interface, where the communication interface is coupled to the processor, and the processor is configured to run a program or instructions, to implement the method according to the first aspect or the second aspect.

In the embodiments of this application, the information processing method includes: obtaining health data of a user; receiving time information sent by an electronic device in a case that a preset condition is met; and sending first information to the electronic device based on the time information, where the preset condition includes at least one of the following: a data volume of the obtained health data being greater than or equal to a preset value, or a first instruction being obtained, where the first instruction is used for instructing to stop obtaining the health data; and in a case that the preset condition is a data volume of the obtained health data being greater than or equal to a preset value, the first information includes: storage position information of health data within a first time range and a data identifier of the health data, where the first time range is a time range corresponding to the time information; or in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained. The health data can be transmitted to the electronic device in segments in advance, instead of starting to be transmitted after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

### BRIEF DESCRIPTION OF THE DRAWINGS

FIG. 1 is a schematic flowchart 1 of an information processing method according to an embodiment of this application;
FIG. 2 is a schematic flowchart 2 of an information processing method according to an embodiment of this application;
FIG. 3 is a schematic diagram of an implementation architecture of an information processing method according to an embodiment of this application;
FIG. 4 is a schematic flowchart of a specific implementation of an information processing method according to an embodiment of this application;
FIG. 5 is a schematic structural diagram 1 of an information processing apparatus according to an embodiment of this application;
FIG. 6 is a schematic structural flowchart 2 of an information processing apparatus according to an embodiment of this application;
FIG. 7 is a schematic structural diagram 1 of a wearable device according to an embodiment of this application;
FIG. 8 is a schematic structural diagram 1 of an electronic device according to an embodiment of this application;
FIG. 9 is a schematic structural diagram 2 of a wearable device according to an embodiment of this application; and
FIG. 10 is a schematic structural diagram 2 of an electronic device according to an embodiment of this application.

### DETAILED DESCRIPTION

The following clearly describes the technical solutions in the embodiments of this application with reference to the accompanying drawings in the embodiments of this application. Apparently, the described embodiments are some but not all of the embodiments of this application. All other embodiments obtained by a person of ordinary skill in the art based on the embodiments of this application fall within the protection scope of this application.

The specification and claims of this application, and terms "first" and "second" are used to distinguish similar objects, but are unnecessarily used to describe a specific sequence or order. It should be understood that the data in such a way are interchangeable in proper circumstances, so that the embodiments of this application can be implemented in other orders than the order illustrated or described herein. Objects distinguished by "first", "second", and the like are usually one type, and the number of objects is not limited. For example, the first object may be one or more than one. In addition, in the specification and the claims, "and/or" represents at least one of the connected objects, and the character "/" generally represents an "or" relationship between the associated objects.

An information processing method provided in the embodiments of this application is described below through specific embodiments and application scenarios thereof with reference to the accompanying drawings.

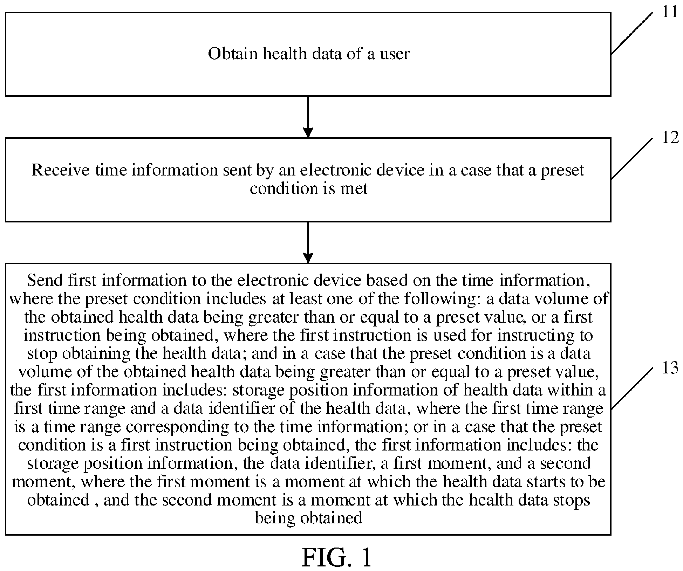

An embodiment of this application provides an information processing method (which may be applied to a wearable device such as a watch). As shown in FIG. 1, the method includes:
Step 11: Obtain health data of a user.

The health data may be sleep data, or the like. This is not limited herein.

Step 12: Receive time information sent by an electronic device in a case that a preset condition is met.

Whether the preset condition is met may be locally monitored, a request is sent to the electronic device in a case that the preset condition is met, and the time information sent by the electronic device is received based on the request. Alternatively, the electronic device monitors whether the preset condition is met, and sends the time information to a wearable device in a case that the preset condition is met.

Step 13: Send first information to the electronic device based on the time information, where the preset condition includes at least one of the following: a data volume of the obtained health data being greater than or equal to a preset value, or a first instruction being obtained, where the first instruction is used for instructing to stop obtaining the health data; and in a case that the preset condition is a data volume of the obtained health data being greater than or equal to a preset value, the first information includes: storage position information of health data within a first time range and a data identifier of the health data, where the first time range is a time range corresponding to the time information; or in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained.

That is, corresponding information is sent to the electronic device based on the met condition.

In the embodiments of this application, the information processing method includes: obtaining health data of a user; receiving time information sent by an electronic device in a case that a preset condition is met; and sending first information to the electronic device based on the time information, where the preset condition includes at least one of the following: a data volume of the obtained health data being greater than or equal to a preset value, or a first instruction being obtained, where the first instruction is used for instructing to stop obtaining the health data; and in a case that the preset condition is a data volume of the obtained health data being greater than or equal to a preset value, the first information includes: storage position information of health data within a first time range and a data identifier of the health data, where the first time range is a time range corresponding to the time information; or in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained. The health data can be transmitted to the electronic device in segments in advance, instead of starting to be transmitted after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

The obtaining health data of a user includes: obtaining first health data and the difference between second health data and third health data; storing the first health data and the difference, where the first health data is health data obtained at the first moment; the second health data is health data obtained at a third moment; and the third health data is health data obtained at a fourth moment, where the third moment is adjacent to the fourth moment, the third moment is within the first moment and the second moment (inclusive), and the fourth moment is within the first moment and the second moment (inclusive).

In this way, storage space can be saved and subsequent data transmission duration can be shortened. The third moment is the first moment, and the fourth moment is between the first moment and the second moment; or the third moment is between the first moment and the second moment, and the fourth moment is between the first moment and the second moment; or the third moment is between the first moment and the second moment, and the fourth moment is the second moment.

Further, after the receiving time information sent by an electronic device, the method further includes: restoring the obtained health data based on the first health data and the difference; determining the health data within the first time range based on the restored health data; and writing the difference corresponding to the health data within the first time range and the first health data into a synchronization file, where different synchronization files correspond to different storage position information.

In this way, the health data within the first time range can be accurately obtained and written into the file.

The determining the health data within the first time range based on the restored health data includes: obtaining the restored health data within a target time range as the health data within the first time range, where the target time range includes: a start moment of the first time range to an end moment of the first time range; or a start moment of the first time range to a moment at which a last item is obtained, the last item is a last item of the obtained health data, and the moment at which a last item is obtained is earlier than the end moment of the first time range.

In this way, all health data within the target time range can be accurately obtained.

Further, in a case that the preset condition is a data volume of the obtained health data being greater than or equal to a preset value, after the sending first information to the electronic device based on the time information, the method further includes: recalculating a data volume of health data that continues to be obtained, and returning to the receiving time information sent by an electronic device in a case that a preset condition is met.

In this way, the health data can be transmitted to the electronic device in segments in this solution.

The first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and in a case that the preset condition is a first instruction being obtained, and the first instruction is an instruction triggered before the target event ends, after the sending first information to the electronic device based on the time information, the method further includes: recalculating a data volume of health data that continues to be obtained, and returning to the receiving time information sent by an electronic device in a case that a preset condition is met.

In this way, it can be ensured that the health data is still normally processed in a scenario in which collection of the health data temporarily ends midway (for example, the user wakes up midway).

Further, in a case that the preset condition is a first instruction being obtained, after the sending first information to the electronic device based on the time information, the method further includes: obtaining the time difference between a fifth moment and the second moment; and sending the fifth moment to the electronic device in a case that the time difference is greater than a retransmission threshold, where the fifth moment is an actual moment at which a target event corresponding to the health data ends, and the second moment is a reference moment at which the target event ends.

In this way, accuracy of the end moment of the feedback to the electronic device can be ensured. The "reference moment" may be a temporary end moment given after the target event ends, for example, a user immediately views a sleep time temporarily given by a watch after waking up.

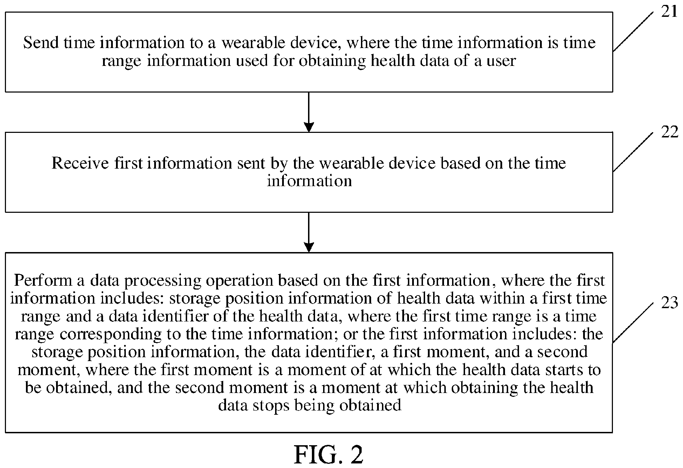

An embodiment of this application further provides an information processing method (which may be applied to an electronic device such as a mobile phone). As shown in FIG. 2, the method includes:
Step 21: Send time information to a wearable device, where the time information is time range information used for obtaining health data of a user.

The health data may be sleep data, or the like. This is not limited herein.

Step 21 may include: sending the time information to the wearable device in a case that a preset condition is met, where the preset condition includes at least one following: a data volume of health data obtained by the wearable device being greater than or equal to a preset value, or the wearable device (or the electronic device) obtaining a first instruction, where the first instruction is used for instructing to stop obtaining the health data.

Step 22: Receive first information sent by the wearable device based on the time information.

The first information may be: corresponding information sent by the wearable device to the electronic device based on the met condition.

Step 23: Perform a data processing operation based on the first information, where the first information includes: storage position information of health data within a first time range and a data identifier of the health data, where the first time range is a time range corresponding to the time information; or the first information includes: the storage position information, the data identifier, a first moment, and a second moment, where the first moment is a moment at which the health data starts to be obtained, and the second moment is a moment at which the health data stops being obtained.

The data processing operation may include: storing data and updating the time information; or storing data for analysis. This is not limited herein.

In the embodiments of this application, the information processing method includes: sending time information to a wearable device, where the time information is time range information used for obtaining health data of a user; receiving first information sent by the wearable device based on the time information; and performing a data processing operation based on the first information, where the first information includes: storage position information of health data within a first time range and a data identifier of the health data, where the first time range is a time range corresponding to the time information; or the first information includes: the storage position information, the data identifier, a first moment, and a second moment, where the first moment is a moment at which the health data starts to be obtained, and the second moment is a moment at which the health data stops being obtained. The wearable device can transmit the health data to the electronic device in segments in advance, instead of starting to transmit the health data after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

The performing a data processing operation based on the first information includes: obtaining health data of a user within the first time range based on the storage position information and the data identifier and storing the health data in a case that the first information includes the storage position information and the data identifier; or obtaining health data of a user within the first time range based on the storage position information and the data identifier and storing the health data in a case that the first information includes the storage position information, the data identifier, the first moment, and the second moment; splicing all the obtained health data corresponding to the data identifier; and analyzing the spliced health data based on the first moment and the second moment.

In this way, normal processing of the health data transmitted in segments can be ensured.

In this embodiment of this application, the health data includes: first health data and the difference between second health data and third health data, where the first health data is health data obtained at the first moment; the second health data is health data obtained at a third moment; and the third health data is health data obtained at a fourth moment, where the third moment is adjacent to the fourth moment, the third moment is within the first moment and the second moment, and the fourth moment is within the first moment and the second moment; and the analyzing the spliced health data based on the first moment and the second moment includes: restoring the spliced health data based on the first health data and the difference; and analyzing the restored health data based on the first moment and the second moment.

In this way, data transmission duration can be shortened. The third moment is the first moment, and the fourth moment is between the first moment and the second moment; or the third moment is between the first moment and the second moment, and the fourth moment is between the first moment and the second moment; or the third moment is between the first moment and the second moment, and the fourth moment is the second moment.

The first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and the analyzing the spliced health data based on the first moment and the second moment includes: analyzing the spliced health data based on the first moment and a target moment, where the target moment is a moment at which a target instruction is triggered, and the target instruction is a first instruction triggered when the target event ends; and the performing a data processing operation based on the first information further includes: obtaining a health data of a user within the first time range based on the storage position information and the data identifier and storing the health data in a case that the first instruction is an instruction triggered before the target event ends.

In this way, it can be ensured that the health data is still normally processed in a scenario in which collection of the health data temporarily ends midway (for example, the user wakes up midway).

Further, the analyzing the spliced health data based on the first moment and the second moment includes: in a case that a fifth moment sent by the wearable device is not received after the first information is received, analyzing the spliced health data based on the first moment and the second moment; and in a case that the fifth moment is received after the first information is received, analyzing the spliced health data based on the first moment and the fifth moment, where the fifth moment is an actual moment at which a target event corresponding to the health data ends, and the second moment is a reference moment at which the target event ends.

In this way, accuracy of an analysis result can be ensured. The "reference moment" may be a temporary end moment given after the target event ends, for example, a user immediately views a sleep time temporarily given by a watch after waking up.

The sending time information to a wearable device includes: using an end moment at which the health data is obtained in a historical record as a start moment at which the health data is obtained; determining, based on a system moment, an end moment at which the health data is obtained, where the end moment is a moment before the system moment or the system moment; obtaining, based on the start moment and the end moment, time information for obtaining health data of a user; and sending the time information to the wearable device.

In this way, the time information can be variously determined. Specifically, the sending time information to a wearable device may include: using an end moment at which the health data is obtained (last time) in a historical record as a start moment at which the health data is obtained (this time); determining the end moment at which the health data is obtained (this time) based on a system (current) moment, where the end moment (this time) is a moment before the system (current) moment or the system (current) moment; and obtaining time information for obtaining the health data of the user based on the start moment (at which the health data is obtained this time) and the end moment; and sending the time information to the wearable device.

The information processing method provided in the embodiments of this application is described below by using an example in which the wearable device is a watch, the electronic device is a mobile phone, and the health data is sleep data (which may be specifically ventricular beat interval RRI data).

For the foregoing technical problem, the embodiments of this application provide an information processing method, which may be specifically implemented as a sleep data synchronization optimization method for a smart watch. First, the watch synchronizes RRI data to a mobile phone side in segments in advance in a sleep process of the user. Next, after the user wakes up, fall asleep time (corresponding to the foregoing first moment) and wake-up time (corresponding to the foregoing second moment) of an entire period of sleep are synchronized to the mobile phone. Finally, after processing the RRI data, the fall asleep fall asleep time, and the wake-up time, the mobile phone may analyze complete sleep data (corresponding to the foregoing analyzing the spliced health data based on the first moment and the second moment) including sleep stages and scores through a CPC (cardiopulmonary coupling analysis) algorithm, and send the health data back to the watch. In this way, a data synchronization time is greatly reduced, so that the watch and the mobile phone can display complete sleep data to the user faster, and provide a better user experience. Specifically, the solution provided in this embodiment of this application may include the following two parts.

Part 1: A basic structure of a sleep function.

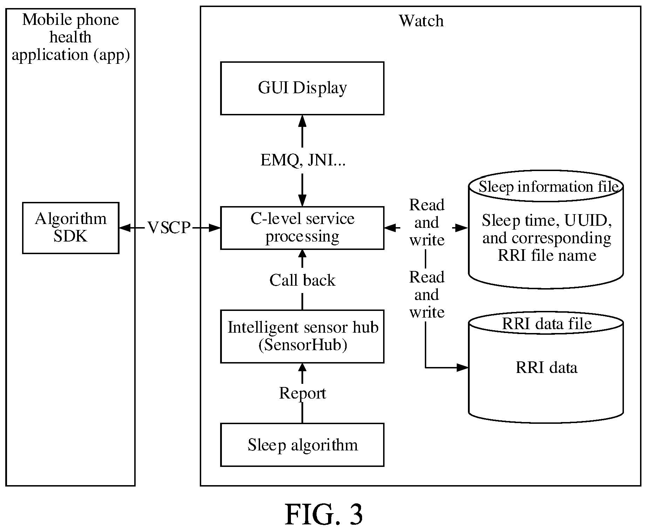

As shown in FIG. 3, a main structure of the sleep function includes: a watch and a mobile phone health application (app). The watch further includes a sleep algorithm, a smart sensor hub (SensorHub), a service C-side (which may also be referred to as a service processing side, for C-level service processing), and a sleep user interface (User Interface, UI, for graphics user interface (Graphics User Interface, GUI) display). The watch sleep algorithm is responsible for sleep monitoring and data reporting, the SensorHub is responsible for distributing data reported by the algorithm, the service C-side performs some service logic processing, the UI side performs data display, and the health app is responsible for analyzing and displaying the sleep data. SDK in FIG. 3 represents software development kit (Software Development Kit); VSCP represents a Bluetooth transmission protocol; GUI represents graphics user interface; and EMQ and JNI represent data exchange interfaces.

Sleep-related information is stored in a file. A sleep information file may include all sleep data information of the watch within 7 days, including:
a sleep time: fall asleep and wake-up time timestamps during this period of sleep. The time before waking up corresponds to 0;
a universally unique identifier (Universally Unique Identifier, UUID): corresponding to the foregoing data identifier, a unique identifier character of a period of sleep; and
a file name corresponding to the RRI: each period of sleep has a separate RRI data file, used for storing RRI data of the period of sleep. This file has a large data volume.

Part 2: A segmented transmission procedure.

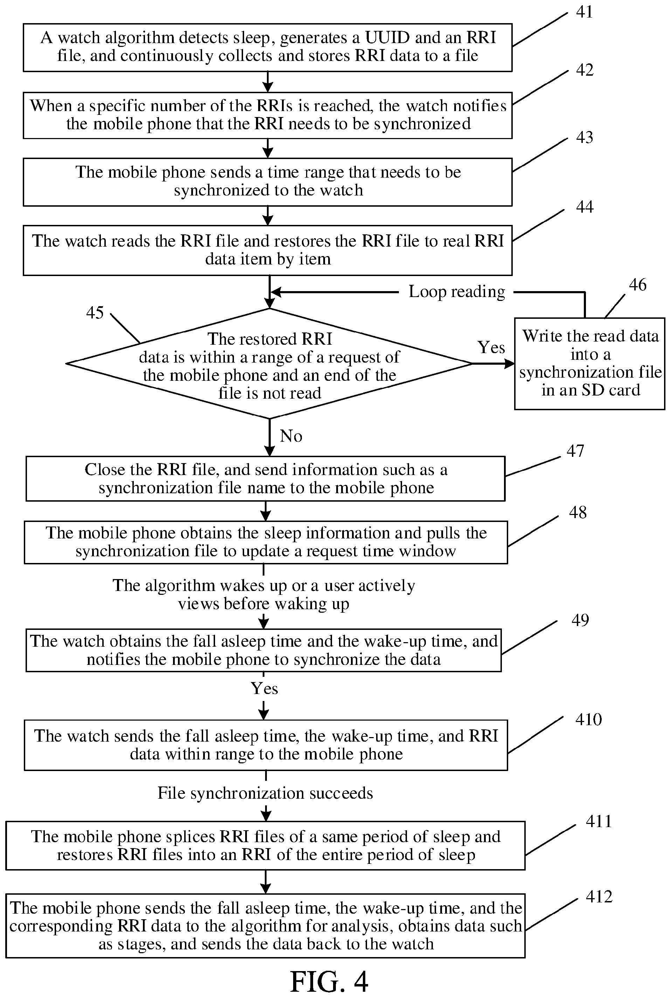

The segmented transmission may be understood as follows: A specific procedure of dividing an RRI of a period of sleep into a plurality of segments and transmitting the RRIs to the mobile phone may be shown in FIG. 4. The procedure includes the following steps:
Step 41: A watch algorithm detects sleep, generates a UUID and an RRI file, and continuously collects and stores RRI data to a file.

Specifically, it may be that: When detecting sleep, the watch sleep algorithm generates a UUID for the period of sleep as a unique identifier (corresponding to the foregoing data identifier), then generates an RRI file for storing the period of sleep, and stores a file name and the UUID to the sleep information file.

In addition, the watch continuously collects the RRI data in a sleep process, performs the difference between two pieces of RRI data (at any two adjacent moments), and stores the first item of the entire period of sleep and the difference to an RRI data file (that is, stores the first item and the obtained difference). In this way, the volume of the RRI file can be reduced, and the subsequent synchronization time can be reduced. Corresponding to the foregoing obtaining the first health data and the difference between the second health data and the third health data, the first health data and the difference are stored.

Step 42: When a specific number of the RRIs is reached, the watch notifies the mobile phone that the RRI needs to be synchronized.

Specifically, it may be that: The watch counts the number of collected RRIs in the sleep process, and notifies the mobile phone app to perform synchronization (an agreed instruction may be sent for notification) when a certain number is reached. This corresponds to that the preset condition includes: a data volume of the obtained health data being greater than or equal to a preset value. For the determining of the preset value, for example, Because the RRI can be used to generate staging data for more than 3 hours of sleep, the number of RRIs of 3/2 = 1.5 hours (this is related to the algorithm, and may be an experience value or determined based on the algorithm) is used. In other words, the mobile phone is notified each time a data volume of about 8 KB is generated.

Step 43: The mobile phone sends a time range that needs to be synchronized to the watch (corresponding to the foregoing sending time information to the wearable device).

Specifically, it may be that: After receiving a message from the watch, the mobile phone uses end time of the last synchronization as start time, advances current time of this synchronization by 5 min as end time, and sends the synchronization time range (corresponding to the foregoing first time range) to the watch. This corresponds to the foregoing using an end moment at which the health data is obtained in a historical record as a start moment at which the health data is obtained; determining, based on a system moment, an end moment at which the health data is obtained, where the end moment is a moment before the system moment; obtaining, based on the start moment and the end moment, time information for obtaining health data of a user; and sending the time information to the wearable device.

During the first synchronization, the first start time may be determined based on a storage cycle. For example, if the cycle is 7 days, the first start time is: a moment corresponding to previous 7 days when the watch sends the message, or a moment corresponding to previous 7 days when the mobile phone receives the message, or a moment 7 days ago corresponding to the current time. This is not limited thereto.

Reason for advancing by 5 min: In terms of power consumption, the watch algorithm does not report the RRI in real time, but reporting the RRI to the application (the service C-side) after caching for about 1 min. Therefore, a cut-off time during a request of the mobile phone is generally advanced by 5 min, to avoid missing an RRI that the algorithm has not synchronized in time during two adjacent synchronizations.

Step 44: The watch reads the RRI file and restores the RRI file to real RRI data item by item.

Specifically, it may be that: After receiving the time range from the mobile phone, the watch opens and reads the RRI file. Because the difference is performed on data during previous storage, each time an item is read, the item is re-accumulated and restored to the RRI data. This corresponds to the foregoing restoring the obtained health data based on the first health data and the difference.

Herein, "accumulation and restoration" is intended to determine whether data is within the time range, and data actually stored in the synchronization file is the first item and the difference, so that a transmission amount can be reduced.

Step 45: The restored RRI data is within a range of a request of the mobile phone and an end of the file is not read.

Specifically, it may be that: After the watch reads and restores the RRI data, logical determining is performed. If the data is within a sending range of the mobile phone and an end of the RRI file is not read, go to step 46 for writing and loop reading; or otherwise, go to step 47 to end the reading. This corresponds to the foregoing obtaining the restored health data in a target time range as the health data within the first time range.

Step 46: Write the read data into a synchronization file in a secure digital card (Secure Digital Card, SD Card).

Specifically, it may be that: The watch writes the RRI data into an SD Card path. If it is the first writing of this synchronization, a temporary file may be generated, otherwise, the file is added after a previous file. Then, go back to step 44 to loop reading the RRI and write the RRI into the synchronization file. This corresponds to the foregoing writing the difference corresponding to the health data within the first time range and the first health data into a synchronization file. Different synchronization files correspond to different storage position information.

The synchronization file may be stored in the watch. Regarding the "RRI data", the watch writes the data into the synchronization file while reading, which ends when the time goes out of the range or when the RRI file ends.

Step 47: Close the RRI file, and send information such as a synchronization file name to the mobile phone.

Specifically, it may be that: The watch writes all data that meets this synchronization condition into the synchronization file, closes the RRI file and ends reading, and then sends the sleep information such as the synchronization file name and the UUID to the mobile phone. This corresponds to the foregoing sending first information to the electronic device based on the time information.

Step 48: The mobile phone obtains the sleep information and pulls the synchronization file to update a request time window.

Specifically, it may be that: The mobile phone receives the synchronization information, pulls and stores the synchronization file, updates the time window in which synchronization is requested (that is, updates the foregoing first time range), and then waits for the next synchronization.

Step 49: The watch obtains the fall asleep time and the wake-up time, and notifies the mobile phone to synchronize the data.

Specifically, It may be that: Each time the collected RRI reaches a predetermined number, the watch repeatedly performs step 42 to step 48 (corresponding to the foregoing recalculating a data volume of health data that continues to be obtained, and returning to the receiving time information sent by an electronic device in a case that a preset condition is met), until after the algorithm wakes up or when the user actively enters the watch and/or the mobile phone to view the sleep, the watch obtains and stores the fall asleep time and the wake-up time, and then notifies the mobile phone that synchronization after waking up needs to be performed (synchronization of the RRI data, the sleep time, and the like; and this corresponds to that the preset condition includes: a first instruction being obtained).

Herein, for some special scenarios, there are some processing policies:
(1) Midway wake up processing (whether the user wakes up midway may be determined based on whether the user uses the mobile phone or the watch, and whether the user actually wakes up finally is determined based on the algorithm).
   If the user continues to fall asleep after being awake in the sleep process and viewing the watch, the algorithm does not wake up in this case, but temporarily displays a fall asleep time and a wake-up time to the user (corresponding to that the first instruction is an instruction triggered before a target event corresponding to the health data ends, and the second moment is a moment at which the first instruction is triggered), and then resets a flag of the number of RRIs transmitted in segments (that is, recalculating whether the RRI data reaches a threshold) and notifies the mobile phone for synchronization. After the user falls asleep, a subsequent RRI (data) is transmitted in segments until the user actually wakes up (corresponding to that the first instruction is an instruction triggered when the target event ends, and the second moment is a moment at which the first instruction is triggered). This corresponds to that, in a case that the preset condition is a first instruction being obtained, and the first instruction is an instruction triggered before the target event ends, after the sending first information to the electronic device based on the time information, the method further includes: recalculating a data volume of health data that continues to be obtained, and returning to the receiving time information sent by an electronic device in a case that a preset condition is met.
(2) Time backward processing:
   If the user views the watch immediately after waking up, a sleep time is also temporarily displayed and synchronized. However, after a while, the algorithm still outputs a real fall asleep time and a real wake-up time. For example, if it is determined that the wake-up time is backward by more than 5 min (that is, the time given by the algorithm is earlier than the temporary time given by the watch), the sleep time is additionally synchronized to the mobile phone. For example, if the wake-up time is backward by less than 5 min (corresponding to the foregoing retransmission threshold), the time is not updated. This corresponds to the foregoing obtaining a time difference between a fifth moment and the second moment; sending the fifth moment to the electronic device in a case that the time difference is greater than a retransmission threshold, where the fifth moment is an actual moment at which a target event corresponding to the health data ends, and the second moment is a reference moment at which the target event ends.

Step 410: The watch sends the fall asleep time, the wake-up time, and RRI data within the range to the mobile phone.

Specifically, it may be that: after receiving a wake-up message, the mobile phone updates an end time of the request (an end time of the last piece of data) to the current time to request the watch for synchronization (when waking up, the algorithm outputs all RRIs, so that there is no need to advance by 5 min). This corresponds to the foregoing using an end moment at which the health data is obtained in a historical record as a start moment at which the health data is obtained; determining, based on a system moment, an end moment at which the health data is obtained, where the end moment is the system moment; obtaining, based on the start moment and the end moment, time information for obtaining health data of a user; and sending the time information to the wearable device.

The watch sends the fall asleep time, the wake-up time, and the RRI within a condition range to the mobile phone (obtained by repeating step 44 to step 47). This corresponds to that in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained.

Step 411: The mobile phone splices RRI files of a same period of sleep and restores the RRI files into an RRI of the entire period of sleep.

Specifically, it may be that: after obtaining the sleep time and the last RRI, the mobile phone splices all synchronization files (in a file obtaining order) of the same UUID and restores the files into complete RRI data. This corresponds to the foregoing obtaining health data of a user within the first time range based on the storage position information and the data identifier and storing the health data in a case that the first information includes the storage position information, the data identifier, the first moment, and the second moment; splicing all the obtained health data corresponding to the data identifier; and analyzing the spliced health data based on the first moment and the second moment.

In this solution, it may be that: A piece of RRI data corresponds to a synchronization file. This is not limited thereto.

Step 412: The mobile phone sends the fall asleep time, the wake-up time, and the corresponding RRI data to the algorithm for analysis, obtains data such as stages, and sends the data back to the watch.

Specifically, it may be that: the mobile phone cuts and arranges the RRI data of the entire period of sleep based on the fall asleep time and the wake-up time, and enters the algorithm SDK for analysis. Then, detailed information such as sleep stages (namely, shallow sleep, deep sleep, rapid eye movement sleep, and the like) and scores is obtained and sent back to the watch. It may be understood that the mobile phone feeds back an analysis result for the "spliced health data" to the watch.

From the above, this solution provides a sleep data optimization solution based on a smart watch. When this solution is applied to sleep synchronization, a delay time can be reduced by about 80%. Particularly, for a mobile phone with a slow Bluetooth transmission rate, reducing a synchronization time can reduce user perception for data synchronization, and greatly improve user experience.

It is noted that, the segmented synchronization of the sleep data in this solution can also be used for synchronization of other health data. Details are not described herein again.

It should be noted that, in the information processing method provided in the embodiments of this application, an execution body may be an information processing apparatus, or a control module configured to perform the information processing method in the information processing apparatus. In the embodiments of this application, an example in which the information processing apparatus performs the information processing method is used to describe the information processing apparatus provided in the embodiments of this application.

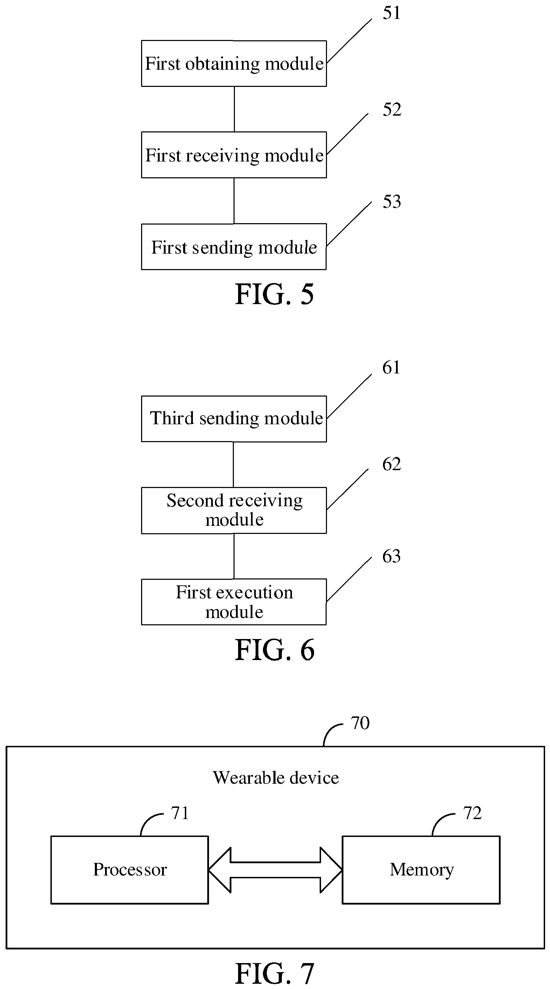

An embodiment of this application provides an information processing apparatus. As shown in FIG. 5, the apparatus includes:
a first obtaining module 51, configured to obtain health data of a user;
a first receiving module 52, configured to receive time information sent by an electronic device in a case that a preset condition is met; and
a first sending module 53, configured to send first information to the electronic device based on the time information, where
the preset condition includes at least one of the following: a data volume of the obtained health data being greater than or equal to a preset value, or a first instruction being obtained, where the first instruction is used for instructing to stop obtaining the health data; and
in a case that the preset condition is a data volume of the obtained health data being greater than or equal to a preset value, the first information includes: storage position information of health data within a first time range and a data identifier of the health data, where the first time range is a time range corresponding to the time information; or
in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained.

The obtaining health data of a user includes: obtaining first health data and the difference between second health data and third health data; storing the first health data and the difference, where the first health data is health data obtained at the first moment; the second health data is health data obtained at a third moment; and the third health data is health data obtained at a fourth moment, where the third moment is adjacent to the fourth moment, the third moment is within the first moment and the second moment, and the fourth moment is within the first moment and the second moment.

Further, the information processing apparatus further includes:
a first processing module, configured to restore the obtained health data based on the first health data and the difference after the time information sent by the electronic device is received; a first determining module, configured to: determine health data within the first time range based on the restored health data; and write the difference corresponding to the health data within the first time range and the first health data into a synchronization file, where different synchronization files correspond to different storage position information.

The determining the health data within the first time range based on the restored health data includes: obtaining the restored health data within a target time range as the health data within the first time range, where the target time range includes: a start moment of the first time range to an end moment of the first time range; or a start moment of the first time range to a moment at which a last item is obtained, the last item is a last item of the obtained health data, and the moment at which a last item is obtained is earlier than the end moment of the first time range.

Further, the information processing apparatus further includes:
a second processing module, configured to: in a case that the preset condition is a data volume of the obtained health data being greater than or equal to a preset value, after the first information is sent to the electronic device based on the time information, recalculate a data volume of health data that continues to be obtained, and return to the receiving time information sent by an electronic device in a case that a preset condition is met.

The first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and the information processing apparatus further includes: a third processing module, configured to: in a case that the preset condition is a first instruction being obtained, and the first instruction is an instruction triggered before the target event ends, after the first information is sent to the electronic device based on the time information, recalculate a data volume of health data that continues to be obtained, and return to the receiving time information sent by an electronic device in a case that a preset condition is met.

Further, the information processing apparatus further includes:
a second obtaining module, configured to: in a case that the preset condition is a first instruction being obtained, after the first information is sent to the electronic device based on the time information, obtain a time difference between a fifth moment and the second moment; and a second sending module, configured to send the fifth moment to the electronic device in a case that the time difference is greater than a retransmission threshold, where the fifth moment is an actual moment at which a target event corresponding to the health data ends, and the second moment is a reference moment at which the target event ends.

In the embodiments of this application, the information processing apparatus obtains health data of a user; receives time information sent by an electronic device in a case that a preset condition is met; and sends first information to the electronic device based on the time information, where the preset condition includes at least one of the following: a data volume of the obtained health data being greater than or equal to a preset value, or a first instruction being obtained, where the first instruction is used for instructing to stop obtaining the health data; and in a case that the preset condition is a data volume of the obtained health data being greater than or equal to a preset value, the first information includes: storage position information of health data within a first time range and a data identifier of the health data, where the first time range is a time range corresponding to the time information; or in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained. The health data can be transmitted to the electronic device in segments in advance, instead of starting to be transmitted after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

The information processing apparatus in the embodiments of this application may be an apparatus, or may be a component, an integrated circuit, or a chip in a wearable device. The apparatus may be a mobile electronic device, or may be a non-mobile electronic device. For example, the mobile electronic device may be a mobile phone, a tablet computer, a laptop computer, a handheld computer, an in-vehicle electronic device, a wearable device, an ultra-mobile personal computer (ultra-mobile personal computer, UMPC), a netbook, or a personal digital assistant (personal digital assistant, PDA), and the non-mobile electronic device may be a server, a network attached storage (Network Attached Storage, NAS), a personal computer (personal computer, PC), a television (television, TV), a teller machine, or a self-service machine. This is not specifically limited in the embodiments of this application.

The information processing apparatus in the embodiments of this application may be an apparatus having an operating system. The operating system may be an Android (Android) operating system, may be an iPhone operating system (iPhone Operating System, iOS), or may be another possible operating system. This is not specifically limited in the embodiments of this application.

The information processing apparatus provided in the embodiments of this application can implement all processes implemented by the method embodiments of FIG. 1, FIG. 3, and FIG. 4. To avoid repetition, details are not described herein again.

An embodiment of this application further provides an information processing apparatus. As shown in FIG. 6, the apparatus includes:
a third sending module 61, configured to send time information to a wearable device, where the time information is time range information used for obtaining health data of a user;
a second receiving module 62, configured to receive first information sent by the wearable device based on the time information; and
a first execution module 63, configured to perform a data processing operation based on the first information, where
the first information includes: storage position information of health data within a first time range and a data identifier of the health data, where the first time range is a time range corresponding to the time information; or
the first information includes: the storage position information, the data identifier, a first moment, and a second moment, where the first moment is a moment at which the health data starts to be obtained, and the second moment is a moment at which the health data stops being obtained.

The performing a data processing operation based on the first information includes: obtaining health data of a user within the first time range based on the storage position information and the data identifier and storing the health data in a case that the first information includes the storage position information and the data identifier; or obtaining health data of a user within the first time range based on the storage position information and the data identifier and storing the health data in a case that the first information includes the storage position information, the data identifier, the first moment, and the second moment; splicing all the obtained health data corresponding to the data identifier; and analyzing the spliced health data based on the first moment and the second moment.

In this embodiment of this application, the health data includes: first health data and the difference between second health data and third health data, where the first health data is health data obtained at the first moment; the second health data is health data obtained at a third moment; and the third health data is health data obtained at a fourth moment, where the third moment is adjacent to the fourth moment, the third moment is within the first moment and the second moment, and the fourth moment is within the first moment and the second moment; and the analyzing the spliced health data based on the first moment and the second moment includes: restoring the spliced health data based on the first health data and the difference; and analyzing the restored health data based on the first moment and the second moment.

The first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and the analyzing the spliced health data based on the first moment and the second moment includes: analyzing the spliced health data based on the first moment and a target moment, where the target moment is a moment at which a target instruction is triggered, and the target instruction is a first instruction triggered when the target event ends; and the performing a data processing operation based on the first information further includes: obtaining a health data of a user within the first time range based on the storage position information and the data identifier and storing the health data in a case that the first instruction is an instruction triggered before the target event ends.

In this embodiment of this application, the analyzing the spliced health data based on the first moment and the second moment includes: in a case that a fifth moment sent by the wearable device is not received after the first information is received, analyzing the spliced health data based on the first moment and the second moment; and in a case that the fifth moment is received after the first information is received, analyzing the spliced health data based on the first moment and the fifth moment, where the fifth moment is an actual moment at which a target event corresponding to the health data ends, and the second moment is a reference moment at which the target event ends.

The sending time information to a wearable device includes: using an end moment at which the health data is obtained in a historical record as a start moment at which the health data is obtained; determining, based on a system moment, an end moment at which the health data is obtained, where the end moment is a moment before the system moment or the system moment; obtaining, based on the start moment and the end moment, time information for obtaining health data of a user; and sending the time information to the wearable device.

In this embodiment of this application, the information processing apparatus sends time information to a wearable device, where the time information is time range information used for obtaining health data of a user; receives first information sent by the wearable device based on the time information; and performs a data processing operation based on the first information, where the first information includes: storage position information of health data within a first time range and a data identifier of the health data, where the first time range is a time range corresponding to the time information; the first information includes: the storage position information, the data identifier, a first moment, and a second moment, where the first moment is a moment at which the health data starts to be obtained, and the second moment is a moment at which the health data stops being obtained. The wearable device can transmit the health data to the electronic device in segments in advance, instead of starting to transmit the health data after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

The information processing apparatus in the embodiments of this application may be an apparatus, or may be a component, an integrated circuit, or a chip in a terminal. The apparatus may be a mobile electronic device, or may be a non-mobile electronic device. For example, the mobile electronic device may be a mobile phone, a tablet computer, a laptop computer, a handheld computer, an in-vehicle electronic device, a wearable device, an ultra-mobile personal computer (ultra-mobile personal computer, UMPC), a netbook, or a personal digital assistant (personal digital assistant, PDA), and the non-mobile electronic device may be a server, a network attached storage (Network Attached Storage, NAS), a personal computer (personal computer, PC), a television (television, TV), a teller machine, or a self-service machine. This is not specifically limited in the embodiments of this application.

The information processing apparatus in the embodiments of this application may be an apparatus having an operating system. The operating system may be an Android (Android) operating system, may be an iOS operating system, or may be another possible operating system. This is not specifically limited in the embodiments of this application.

The information processing apparatus provided in the embodiments of this application can implement all processes implemented by the method embodiments of FIG. 2 to FIG. 4. To avoid repetition, details are not described herein again.

Optionally, as shown in FIG. 7, an embodiment of this application further provides a wearable device 70, including a processor 71, a memory 72, and a program or instructions stored on the memory 72 and executable on the processor 71. The program or the instructions, when executed by the processor 71, implement all processes of the foregoing information processing method embodiment of FIG. 1, and can achieve the same technical effects. To avoid repetition, details are not described herein again.

It should be noted that, the wearable device in the embodiments of this application includes the foregoing mobile electronic device and non-mobile electronic device.

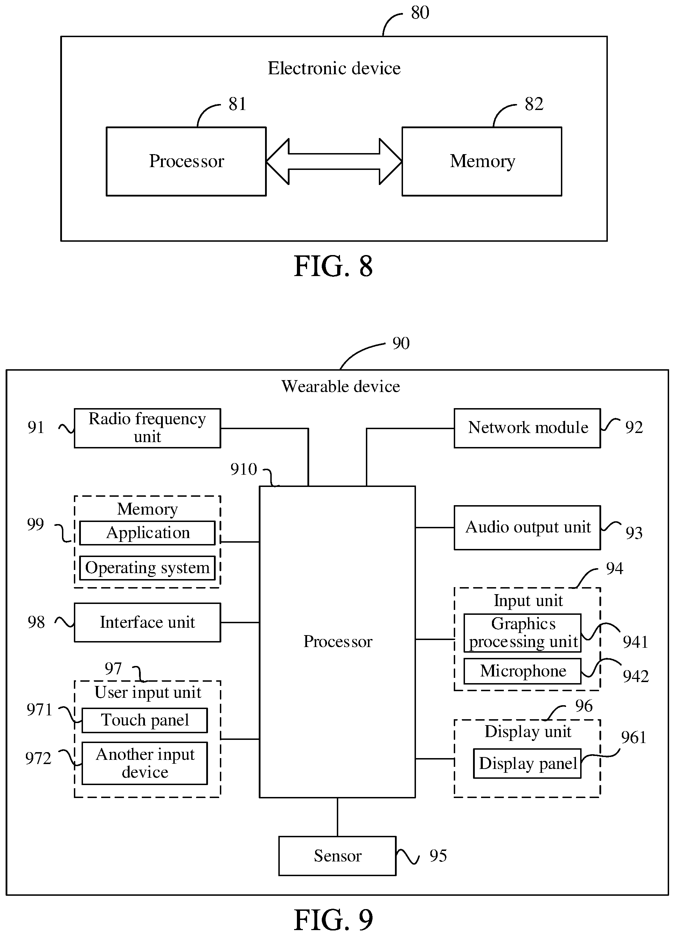

Optionally, as shown in FIG. 8, an embodiment of this application further provides an electronic device 80, including a processor 81, a memory 82, and a program or instructions stored on the memory 82 and executable on the processor 81. The program or the instructions, when executed by the processor 81, implement all processes of the foregoing information processing method embodiment of FIG. 2, and can achieve the same technical effects. To avoid repetition, details are not described herein again.

It should be noted that, the electronic device in the embodiments of this application includes the foregoing mobile electronic device and non-mobile electronic device.

FIG. 9 is a schematic diagram of a hardware structure of a wearable device according to an embodiment of this application.

The wearable device 90 includes, but not limited to: components such as a radio frequency unit 91, a network module 92, an audio output unit 93, an input unit 94, a sensor 95, a display unit 96, a user input unit 97, an interface unit 98, a memory 99, and a processor 910.

A person skilled in the art may understand that the wearable device 90 further includes a power supply (such as a battery) for supplying power to the components. The power supply may logically connect to the processor 910 by using a power supply management system, thereby implementing functions, such as charging, discharging, and power consumption management, by using the power supply management system. A structure of the wearable device shown in FIG. 9 does not constitute a limitation to the wearable device, and the wearable device may include more or fewer components than those shown in the figure, or some components may be combined, or a different component deployment may be used. Details are not described herein again.

The processor 910 is configured to: obtain health data of a user; receive, through the radio frequency unit 91, time information sent by an electronic device in a case that a preset condition is met; and send first information to the electronic device based on the time information through the radio frequency unit 91, where the preset condition includes at least one of the following: a data volume of the obtained health data being greater than or equal to a preset value, or a first instruction being obtained, where the first instruction is used for instructing to stop obtaining the health data; and in a case that the preset condition is a data volume of the obtained health data being greater than or equal to a preset value, the first information includes: storage position information of health data within a first time range and a data identifier of the health data, where the first time range is a time range corresponding to the time information; or in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained.

In the embodiments of this application, the information processing method includes: obtaining health data of a user; receiving time information sent by an electronic device in a case that a preset condition is met; and sending first information to the electronic device based on the time information, where the preset condition includes at least one of the following: a data volume of the obtained health data being greater than or equal to a preset value, or a first instruction being obtained, where the first instruction is used for instructing to stop obtaining the health data; and in a case that the preset condition is a data volume of the obtained health data being greater than or equal to a preset value, the first information includes: storage position information of health data within a first time range and a data identifier of the health data, where the first time range is a time range corresponding to the time information; or in a case that the preset condition is a first instruction being obtained, the first information includes: the storage position information, the data identifier, a first moment, and a second moment, where the first moment is a moment at which the health data starts to be obtained, and the second moment is a moment at which the health data stops being obtained. The health data can be transmitted to the electronic device in segments in advance, instead of starting to be transmitted after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

Optionally, the obtaining health data of a user includes: obtaining first health data and the difference between second health data and third health data; storing the first health data and the difference, where the first health data is health data obtained at the first moment; the second health data is health data obtained at a third moment; and the third health data is health data obtained at a fourth moment, where the third moment is adjacent to the fourth moment, the third moment is within the first moment and the second moment, and the fourth moment is within the first moment and the second moment.

Optionally, the processor 910 is further configured to: restore the obtained health data based on the first health data and the difference after receiving the time information sent by the electronic device; determine the health data within the first time range based on the restored health data; and write the difference corresponding to the health data within the first time range and the first health data into a synchronization file, where different synchronization files correspond to different storage position information.

Optionally, the determining the health data within the first time range based on the restored health data includes: obtaining the restored health data within a target time range as the health data within the first time range, where the target time range includes: a start moment of the first time range to an end moment of the first time range; or a start moment of the first time range to a moment at which a last item is obtained, the last item is a last item of the obtained health data, and the moment at which a last item is obtained is earlier than the end moment of the first time range.

Optionally, the processor 910 is further configured to: in a case that the preset condition is a data volume of the obtained health data being greater than or equal to a preset value, after the first information is sent to the electronic device through the radio frequency unit 91 based on the time information, recalculate a data volume of health data that continues to be obtained, and return to the receiving, through the radio frequency unit 91, time information sent by an electronic device in a case that a preset condition is met.

Optionally, the first instruction is an instruction triggered before a target event corresponding to the health data ends, the second moment is a moment at which the first instruction is triggered, or the first instruction is an instruction triggered when the target event ends, and the second moment is a moment at which the first instruction is triggered.

The processor 910 is further configured to: in a case that the preset condition is a first instruction being obtained, and the first instruction is an instruction triggered before the target event ends, after the first information is sent to the electronic device through the radio frequency unit 91 based on the time information, recalculate a data volume of health data that continues to be obtained, and return to the receiving, through the radio frequency unit 91, time information sent by an electronic device in a case that a preset condition is met.

Optionally, the processor 910 is further configured to: in a case that the preset condition is a first instruction being obtained, after sending the first information to the electronic device based on the time information through the radio frequency unit 91, obtain a time difference between a fifth moment and the second moment; and send the fifth moment to the electronic device through the radio frequency unit 91 in a case that the time difference is greater than a retransmission threshold, where the fifth moment is an actual moment at which a target event corresponding to the health data ends, and the second moment is a reference moment at which the target event ends.

This solution can reduce user perception for data synchronization by reducing a data synchronization time, and greatly improve user experience.

It should be understood that, in this embodiment of this application, the input unit 94 may include a graphics processing unit (Graphics Processing Unit, GPU) 941 and a microphone 942. The graphics processing unit 941 performs processing on image data of a static picture or a video that is obtained by an image acquisition apparatus (for example, a camera) in a video acquisition mode or an image acquisition mode. The display unit 96 may include a display panel 961, for example, the display panel 961 may be configured in a form such as a liquid crystal display or an organic light-emitting diode. The user input unit 97 includes a touch panel 971 and another input device 972. The touch panel 971 is also referred to as a touch screen. The touch panel 971 may include two parts: a touch detection apparatus and a touch controller. The another input device 972 may include, but not limited to, a physical keyboard, a functional key (such as a volume control key or a switch key), a track ball, a mouse, and a joystick, and details are not described herein again. The memory 99 may be configured to store a software program and various data, and includes, but not limited to: an application program and an operating system. The processor 910 may integrate an application processor and a modem processor. The application processor mainly processes an operating system, a user interface, an application program, and the like. The modem processor mainly processes wireless communication. It may be understood that the modem processor may not be integrated into the processor 910.

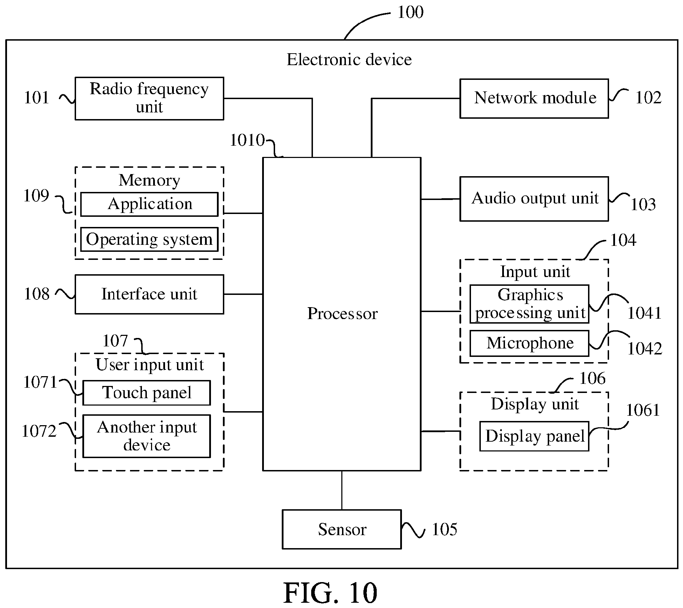

FIG. 10 is a schematic diagram of a hardware structure of an electronic device for implementing the embodiments of this application.

The electronic device 100 includes, but not limited to: components such as a radio frequency unit 101, a network module 102, an audio output unit 103, an input unit 104, a sensor 105, a display unit 106, a user input unit 107, an interface unit 108, a memory 109, and a processor 1010.

A person skilled in the art may understand that the electronic device 100 further includes a power supply (such as a battery) for supplying power to the components. The power supply may logically connect to the processor 1010 by using a power supply management system, thereby implementing functions, such as charging, discharging, and power consumption management, by using the power supply management system. The structure of the electronic device shown in FIG. 10 constitutes no limitation on the electronic device, and the electronic device may include more or fewer components than those shown in the figure, or some components may be combined, or a different component deployment may be used. Details are not described herein again.

The processor 1010 is configured to: send time information to the wearable device through the radio frequency unit 101, where the time information is time range information used for obtaining health data of a user; receive, through the radio frequency unit 101, first information sent by the wearable device based on the time information; and performs a data processing operation based on the first information, where the first information includes: storage position information of health data within a first time range and a data identifier of the health data, where the first time range is a time range corresponding to the time information; the first information includes: the storage position information, the data identifier, a first moment, and a second moment, where the first moment is a moment at which the health data starts to be obtained, and the second moment is a moment at which the health data stops being obtained.

In the embodiments of this application, the information processing method includes: sending time information to a wearable device, where the time information is time range information used for obtaining health data of a user; receiving first information sent by the wearable device based on the time information; and performing a data processing operation based on the first information, where the first information includes: storage position information of health data within a first time range and a data identifier of the health data, where the first time range is a time range corresponding to the time information; the first information includes: the storage position information, the data identifier, a first moment, and a second moment, where the first moment is a moment at which the health data starts to be obtained, and the second moment is a moment at which the health data stops being obtained. The wearable device can transmit the health data to the electronic device in segments in advance, instead of starting to transmit the health data after collection of the health data ends. In this way, a data synchronization time is greatly reduced, relevant analysis on the health data can be displayed to the user faster, a delay time is reduced, and a better user experience is provided. This well resolves the problem of a long delay time in the solution for processing the health data of a user in the related art.

Optionally, the performing a data processing operation based on the first information includes: obtaining health data of a user within the first time range based on the storage position information and the data identifier and storing the health data in a case that the first information includes the storage position information and the data identifier; or obtaining health data of a user within the first time range based on the storage position information and the data identifier and storing the health data in a case that the first information includes the storage position information, the data identifier, the first moment, and the second moment; splicing all the obtained health data corresponding to the data identifier; and analyzing the spliced health data based on the first moment and the second moment.

Optionally, the health data includes: first health data and the difference between second health data and third health data, where the first health data is health data obtained at the first moment; the second health data is health data obtained at a third moment; and the third health data is health data obtained at a fourth moment, where the third moment is adjacent to the fourth moment, the third moment is within the first moment and the second moment, and the fourth moment is within the first moment and the second moment; and the analyzing the spliced health data based on the first moment and the second moment includes: restoring the spliced health data based on the first health data and the difference; and analyzing the restored health data based on the first moment and the second moment.

Optionally, the first instruction is an instruction triggered before a target event corresponding to the health data ends, the second moment is a moment at which the first instruction is triggered, or the first instruction is an instruction triggered when the target event ends, and the second moment is a moment at which the first instruction is triggered. The analyzing the spliced health data based on the first moment and the second moment includes: analyzing the spliced health data based on the first moment and a target moment, where the target moment is a moment at which a target instruction is triggered, and the target instruction is a first instruction triggered when the target event ends; and the performing a data processing operation based on the first information further includes: obtaining a health data of a user within the first time range based on the storage position information and the data identifier and storing the health data in a case that the first instruction is an instruction triggered before the target event ends.

Optionally, the analyzing the spliced health data based on the first moment and the second moment includes: in a case that a fifth moment sent by the wearable device is not received after the first information is received through the radio frequency unit 101, analyzing the spliced health data based on the first moment and the second moment; and in a case that the fifth moment is received after the first information is received through the radio frequency unit 101, analyzing the spliced health data based on the first moment and the fifth moment, where the fifth moment is an actual moment at which a target event corresponding to the health data ends, and the second moment is a reference moment at which the target event ends.

Optionally, the sending time information to a wearable device includes: using an end moment at which the health data is obtained in a historical record as a start moment at which the health data is obtained; determining, based on a system moment, an end moment at which the health data is obtained, where the end moment is a moment before the system moment or the system moment; obtaining, based on the start moment and the end moment, time information for obtaining health data of a user; and sending the time information to the wearable device.

This solution can reduce user perception for data synchronization by reducing a data synchronization time, and greatly improve user experience.

It should be understood that, in this embodiment of this application, the input unit 104 may include a graphics processing unit (Graphics Processing Unit, GPU) 1041 and a microphone 1042. The graphics processing unit 1041 performs processing on image data of a static picture or a video that is obtained by an image acquisition apparatus (for example, a camera) in a video acquisition mode or an image acquisition mode. The display unit 106 may include a display panel 1061, for example, the display panel 1061 may be configured in a form such as a liquid crystal display or an organic light-emitting diode. The user input unit 107 includes a touch panel 1071 and another input device 1072. The touch panel 1071 is also referred to as a touch screen. The touch panel 1071 may include two parts: a touch detection apparatus and a touch controller. The another input device 1072 may include, but not limited to, a physical keyboard, a functional key (such as a volume control key or a switch key), a track ball, a mouse, and a joystick, and details are not described herein again. The memory 109 may be configured to store a software program and various data, and includes, but not limited to: an application program and an operating system. The processor 1010 may integrate an application processor and a modem processor. The application processor mainly processes an operating system, a user interface, an application program, and the like. The modem processor mainly processes wireless communication. It may be understood that the modem processor may not be integrated into the processor 1010.

An embodiment of this application further provides a readable storage medium, having a program or instructions stored therein. The program or instructions, when executed by a processor, implements all processes of the foregoing information processing method embodiment of FIG. 1, and can achieve the same technical effects. To avoid repetition, details are not described herein again.

The processor is a processor in the wearable device in the foregoing embodiments. The readable storage medium includes a computer-readable storage medium, for example, a computer read-only memory (Read-Only Memory, ROM), a random access memory (Random Access Memory, RAM), a magnetic disk, an optical disc, or the like.

An embodiment of this application further provides a readable storage medium, having a program or instructions stored therein. The program or instructions, when executed by a processor, implements all processes of the foregoing information processing method embodiment of FIG. 2, and can achieve the same technical effects. To avoid repetition, details are not described herein again.

The processor is a processor in the electronic device in the foregoing embodiments. The readable storage medium includes a computer-readable storage medium, for example, a computer read-only memory (Read-Only Memory, ROM), a random access memory (Random Access Memory, RAM), a magnetic disk, an optical disc, or the like.

An embodiment of this application further provides a chip, including a processor and a communication interface. The communication interface is coupled to the processor, and the processor is configured to run a program or instructions, to implement all processes of the foregoing information processing method embodiments, and can achieve the same technical effects. To avoid repetition, details are not described herein again.

It should be understood that, the chip mentioned in the embodiments of this application may also be referred to as a system-level chip, a system chip, a chip system, a system on chip, or the like.

It should be noted that, the term "include", "comprise" or any other variation thereof in this specification is intended to cover a non-exclusive inclusion, which specifies the presence of stated processes, methods, objects, or apparatuses, but does not preclude the presence or addition of one or more other processes, methods, objects, or apparatuses. Without more limitations, elements defined by the sentence "including one" does not exclude that there are still other same elements in the processes, methods, objects, or apparatuses. In addition, it should be noted that, the scope of the method and apparatus in the embodiments of this application is not limited to performing functions in the order shown or discussed, and it may further include performing the functions in a substantially simultaneous manner or in reverse order according to the functions involved. For example, the described method may be performed in a different order than described, and various steps may also be added, omitted, or combined. In addition, features described with reference to some examples may be combined in other examples.

Through the descriptions of the foregoing implementations, a person skilled in the art may clearly understand that the method according to the foregoing embodiments may be implemented by means of software and a necessary general hardware platform, and certainly, may alternatively be implemented by hardware, but in many cases, the former manner is a better implementation. Based on such an understanding, the technical solutions in this application essentially or a part that contributes to the related art may be implemented in the form of a computer software product. The computer software product is stored in a storage medium (for example, a ROM/RAM, a magnetic disk, or an optical disc), and includes several instructions for instructing a terminal (which may be a mobile phone, a computer, a server, a network device, or the like) to perform the method described in the embodiments of this application.

The embodiments of this application are described above with reference to the accompanying drawings, but this application is not limited to the foregoing specific embodiments, which are merely illustrative rather than limited. Under the inspiration of this application, a person of ordinary skill in the art may make various variations without departing from the scope of this application and the protection of the claims, and such variations shall fall within the protection of this application.

## Claims

1. An information processing method, comprising:
obtaining health data of a user;
receiving time information sent by an electronic device in a case that a preset condition is met; and
sending first information to the electronic device based on the time information, wherein
the preset condition comprises at least one of the following: a data volume of the obtained health data being greater than or equal to a preset value, or a first instruction being obtained, wherein the first instruction is used for instructing to stop obtaining the health data; and
in a case that the preset condition is a data volume of the obtained health data being greater than or equal to a preset value, the first information comprises: storage position information of health data within a first time range and a data identifier of the health data, wherein the first time range is a time range corresponding to the time information; or
in a case that the preset condition is a first instruction being obtained, the first information comprises: the storage position information, the data identifier, a first moment, and a second moment, wherein the first moment is a moment at which the health data starts to be obtained, and the second moment is a moment at which the health data stops being obtained.

2. The information processing method according to claim 1, the obtaining health data of a user comprises:
obtaining first health data and the difference between second health data and third health data;
storing the first health data and the difference, wherein
the first health data is health data obtained at the first moment;
the second health data is health data obtained at a third moment; and
the third health data is health data obtained at a fourth moment, wherein
the third moment is adjacent to the fourth moment, the third moment is within the first moment and the second moment, and the fourth moment is within the first moment and the second moment.

3. The information processing method according to claim 2, wherein after the receiving time information sent by an electronic device, the method further comprises:
restoring the obtained health data based on the first health data and the difference;
determining the health data within the first time range based on the restored health data; and
writing the difference corresponding to the health data within the first time range and the first health data into a synchronization file, wherein
different synchronization files correspond to different storage position information.

4. The information processing method according to claim 3, wherein the determining the health data within the first time range based on the restored health data comprises:
obtaining the restored health data within a target time range as the health data within the first time range, wherein
the target time range comprises: a start moment of the first time range to an end moment of the first time range; or
a start moment of the first time range to a moment at which a last item is obtained, the last item is a last item of the obtained health data, and the moment at which a last item is obtained is earlier than the end moment of the first time range.

5. The information processing method according to claim 1, wherein in a case that the preset condition is a data volume of the obtained health data being greater than or equal to a preset value, after the sending first information to the electronic device based on the time information, the method further comprises:
recalculating a data volume of health data that continues to be obtained, and returning to the receiving time information sent by an electronic device in a case that a preset condition is met.

6. The information processing method according to claim 1, wherein the first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and
in a case that the preset condition is a first instruction being obtained, and the first instruction is an instruction triggered before the target event ends, after the sending first information to the electronic device based on the time information, the method further comprises:
recalculating a data volume of health data that continues to be obtained, and returning to the receiving time information sent by an electronic device in a case that a preset condition is met.

7. The information processing method according to claim 1, wherein in a case that the preset condition is a first instruction being obtained, after the sending first information to the electronic device based on the time information, the method further comprises:
obtaining a time difference between a fifth moment and the second moment; and
sending the fifth moment to the electronic device in a case that the time difference is greater than a retransmission threshold, wherein
the fifth moment is an actual moment at which a target event corresponding to the health data ends, and the second moment is a reference moment at which the target event ends.

8. An information processing method, comprising:
sending time information to a wearable device, wherein the time information is time range information used for obtaining health data of a user;
receiving first information sent by the wearable device based on the time information; and
performing a data processing operation based on the first information, wherein
the first information comprises: storage position information of health data within a first time range and a data identifier of the health data, wherein the first time range is a time range corresponding to the time information; or
the first information comprises: the storage position information, the data identifier, a first moment, and a second moment, wherein the first moment is a moment at which the health data starts to be obtained, and the second moment is a moment at which the health data stops being obtained.

9. The information processing method according to claim 8, wherein the performing a data processing operation based on the first information comprises:
obtaining health data of a user within the first time range based on the storage position information and the data identifier and storing the health data in a case that the first information comprises the storage position information and the data identifier; or
obtaining health data of a user within the first time range based on the storage position information and the data identifier and storing the health data in a case that the first information comprises the storage position information, the data identifier, the first moment, and the second moment;
splicing all the obtained health data corresponding to the data identifier; and
analyzing the spliced health data based on the first moment and the second moment.

10. The information processing method according to claim 9, wherein the health data comprises: first health data and the difference between second health data and third health data, wherein the first health data is health data obtained at the first moment; the second health data is health data obtained at a third moment; and the third health data is health data obtained at a fourth moment, wherein the third moment is adjacent to the fourth moment, the third moment is within the first moment and the second moment, and the fourth moment is within the first moment and the second moment; and
the analyzing the spliced health data based on the first moment and the second moment comprises:
restoring the spliced health data based on the first health data and the difference; and
analyzing the restored health data based on the first moment and the second moment.

11. The information processing method according to claim 9, wherein a first instruction is an instruction triggered before a target event corresponding to the health data ends, the second moment is a moment at which the first instruction is triggered, or the first instruction is an instruction triggered when the target event ends, and the second moment is a moment at which the first instruction is triggered; and
the analyzing the spliced health data based on the first moment and the second moment comprises:
analyzing the spliced health data based on the first moment and a target moment, wherein the target moment is a moment at which a target instruction is triggered, and the target instruction is a first instruction triggered when the target event ends; and
the performing a data processing operation based on the first information further comprises:
obtaining a health data of a user within the first time range based on the storage position information and the data identifier and storing the health data in a case that the first instruction is an instruction triggered before the target event ends.

12. The information processing method according to claim 9, wherein the analyzing the spliced health data based on the first moment and the second moment comprises:
in a case that a fifth moment sent by the wearable device is not received after the first information is received, analyzing the spliced health data based on the first moment and the second moment; and
in a case that the fifth moment is received after the first information is received, analyzing the spliced health data based on the first moment and the fifth moment, wherein
the fifth moment is an actual moment at which a target event corresponding to the health data ends, and the second moment is a reference moment at which the target event ends.

13. The information processing method according to claim 8, wherein the sending time information to a wearable device comprises:
using an end moment at which the health data is obtained in a historical record as a start moment at which the health data is obtained;
determining, based on a system moment, an end moment at which the health data is obtained, wherein the end moment is a moment before the system moment or the system moment;
obtaining, based on the start moment and the end moment, time information for obtaining health data of a user; and
sending the time information to the wearable device.

14. An information processing apparatus, comprising:
a first obtaining module, configured to obtain health data of a user;
a first receiving module, configured to receive time information sent by an electronic device in a case that a preset condition is met; and
a first sending module, configured to send first information to the electronic device based on the time information, wherein
the preset condition comprises at least one of the following: a data volume of the obtained health data being greater than or equal to a preset value, or a first instruction being obtained, wherein the first instruction is used for instructing to stop obtaining the health data; and
in a case that the preset condition is a data volume of the obtained health data being greater than or equal to a preset value, the first information comprises: storage position information of health data within a first time range and a data identifier of the health data, wherein the first time range is a time range corresponding to the time information; or
in a case that the preset condition is a first instruction being obtained, the first information comprises: the storage position information, the data identifier, a first moment, and a second moment, wherein the first moment is a moment at which the health data starts to be obtained, and the second moment is a moment at which the health data stops being obtained.

15. An information processing apparatus, comprising:
a third sending module, configured to send time information to a wearable device, wherein the time information is time range information used for obtaining health data of a user;
a second receiving module, configured to receive first information sent by the wearable device based on the time information; and
a first execution module, configured to perform a data processing operation based on the first information, wherein
the first information comprises: storage position information of health data within a first time range and a data identifier of the health data, wherein the first time range is a time range corresponding to the time information; or
the first information comprises: the storage position information, the data identifier, a first moment, and a second moment, wherein the first moment is a moment at which the health data starts to be obtained, and the second moment is a moment at which the health data stops being obtained.

16. A wearable device, comprising a processor, a memory, and a program or instructions stored on the memory and executable on the processor, wherein the program or the instructions, when executed by the processor, implement the steps of the information processing method according to any one of claims 1 to 7.

17. An electronic device, comprising a processor, a memory, and a program or instructions stored on the memory and executable on the processor, wherein the program or the instructions, when executed by the processor, implement the steps of the information processing method according to any one of claims 8 to 13.

18. A readable storage medium, having a program or instructions stored therein, wherein the program or the instructions, when executed by a processor, implement the steps of the information processing method according to any one of claims 1 to 7; or
the program or the instructions, when executed by a processor, implement the steps of the information processing method according to any of claims 8 to 13.
